# EUROPEAN PATENT APPLICATION

(11) **EP 3 318 262 A2**
(43) Date of publication of application: **09.05.2018**
(21) Application number: 17198356.2
(22) Date of filing: 25.10.2017
(51) Int. Cl.: A61K 35/00

(54) **COMPOSITION FOR PREVENTING OR TREATING BONE DISEASE, OBESITY AND LIPID-RELATED METABOLIC DISEASE**

(30) Priority: 03.11.2016 KR 20160146009
(71) Applicant: Cell Biotech Co., Ltd., Wolgot-myeon Gyeonggi-do 10003 (KR)
(72) Inventor: CHUNG, Myung Jun, 06544 Seoul (KR)
(74) Representative: Schnappauf, Georg

(57) **Abstract**

The present invention relates to a functional composition for preventing, alleviating or treating bone disease and lipid-related metabolic disease, and more particularly to a composition which comprises a combination of probiotics and a vitamin complex, which is capable of effectively preventing, alleviating or treating bone disease, obesity and lipid-related metabolic disease by synergistic effects on antioxidant activity, a reduction in blood lipid levels and the promotion of *in vivo* absorption of calcium and iron.

## Description

### [Technical Field]

The present invention relates to a functional composition for preventing, alleviating or treating bone disease, obesity and lipid-related metabolic disease.

### [Background Art]

In recent years in Korea, calorie intake has increased due to the Westernization of eating habits along with economic growth, and thus the prevalence of obesity has increased. Specifically, according to 2013 Yearbook of Health and Welfare Statistics, the proportion of obese people among adult over 19 years old is 31.4%, which is 0.5% higher than the 30.9% reported in 2010, and the childhood obesity rate has increased rapidly.

Although obesity itself increases the body weight, makes the body obese, and thus makes life inconvenient, the bigger problem is that obesity increases blood lipid levels to cause arteriosclerosis and heart disease, increases insulin resistance to cause complications such as diabetes, menstrual irregularity and cancer, and causes chronic adult diseases such as hyperlipidemia, hypertension, coronary artery disease and stroke. For this reason, the treatment and prevention of obesity are essential (Lee JH, J. Kor. Soc. Obes., 1:21-24, 1992; Lew EA, Ann. Intern. Med., 103:1024-1029, 1985; Kim KI et al., Korean J. Food Sci. Technol., 35:720-725,2003).

Obesity is known to be caused by genetic factors, environmental factors resulting from Westernized eating habits, psychological factors resulting from stress, and the like, but the exact causes or mechanisms of obesity have not been clearly found.

In the past, it has been recognized that adipocytes merely act to store surplus energy of the human body in the form of triglycerides and to buffer external impact. However, in recent years, adipocytes have been recognized as an endocrine organ that secretes adipocytokines which regulate fasting metabolism and insulin sensitivity. Specifically, adipocytokines such as adiponectin, leptin, resistin, tumor necrosis factor alpha (TNF-α), interleukin-6 (IL-6) and the like are known to play an important role in maintaining homeostasis and regulating energy metabolism (Matsuzawa, Y. et al., Ann. Ny. Acad. Sci., 892:146-154, 1999; Saltiel, A.R., Nat. Med., 7:887-888, 2001).

Particularly, leptin that is produced in obesity genes is proportional to the amount of adipocytes, and thus the serum leptin level of obese people appears to be higher than that of normal-weight people. Furthermore, leptin is produced much more in subcutaneous fat than in visceral fat. Such leptin that is involved in the endocrine function of adipocytes plays an important role in obesity, acts to control eating, energy expenditure and reproductive function, inhibits appetite, and increases heat production through the sympathetic nervous system. In addition, it was found that leptin circulates to the brain, acts on the hypothalamic receptor to inhibit appetite, is released from the brain upon an increase in body fat, and is one of substances that inhibit appetite by stimulating the satiety center in the brain (Mantzoros, C.S., Ann Intern. Med., 130:671-680, 1999; Roemmich, J.N. and A.D., Am. J. Hum. Biol., 11:209-224, 1999).

The goals for treating obesity can be largely classified into two. The first goal is to burn an excess amount of fat to reduce body weight, and the second goal is to improve metabolic imbalance. Currently, the treatment of obesity aims not only at reducing body weight, but also at improving metabolic abnormalities by early eliminating factors that cause cardiovascular diseases. In addition, studies have been actively conducted to inhibit obesity by controlling eating and energy expenditure. Hypothalamus, motor nervous, autonomic nervous and peripheral nervous systems are all involved in regulation of food intake behaviors. Particularly, the hypothalamus of the central nervous system plays an important role in the pathology of obesity. Typical factors that are secreted from the hypothalamus include neuropeptide Y, POMC/CART, melanocortin receptor, norepinephrine, serotonin, and the like. Current strategies for development of obesity therapeutic agents include reduced food intake, a reduction in caloric absorption, promotion of exothermic reactions, regulation of energy metabolism, regulation of signaling through the nervous system, and the like (Mi-Jung Park, Korean J Pediatr 48(2), 2005).

Obesity therapeutic drugs known to date are largely divided according to the mechanism of action into satiety stimulants, fat absorption inhibitors, and antipsychotic appetite suppressants. The most representative drugs for obesity treatment include Xenical™ (Roche Pharmaceuticals, Switzerland), Reductil™ (Abbott, USA), Exolise™ (Arkopharma France) and the like, but have problems in that they cause fatty stool, intestinal gas generation, abdominal bloating, fecal incontinence and the like, and causes adverse effects such as cardiac diseases, respiratory diseases, neurological diseases and the like, and the persistence of efficacy thereof is low.

Accordingly, in order to minimize the adverse effects of artificially synthesized substances as described above, functional substances effective for weight control have been developed from natural substances. Typical examples of such natural substances include green tea, ginseng, Angelica, Chaga mushroom, red ginseng, Actinidia arguta, brown algae, and the like. However, anti-obesity substances extracted from such natural substances have problems in that the effective concentration of components that exhibit efficacy is low and many costs are incurred because the natural substances are cultivated in farmlands or the like.

Meanwhile, osteoporosis characterized by high risk of bone loss and bone fractures is particularly one of the most common diseases in the elderly, and is estimated to affect about one hundred million people in the world. While the incidence of bone fracture in the elderly is generally increasing, therapeutic choices are limited. Currently, antiresorptives (e.g. bisphosphonates, denosumab, hormone therapy) are the most commonly used treatments for osteoporosis. These agents are designed to slow bone remodeling and increase bone density. However, they have been associated with significant side effects including osteonecrosis of the jaw, atypical fractures, atrial fibrillation, and increased risk of stroke or cancer. Anabolic agents may be used to generate new bone in patients with osteoporosis. However, finding anabolic factors that increase bone mass and regulate the balance between osteoblast-mediated bone formation and bone marrow fat accumulation has been challenging. In addition, the only commercially available anabolic agent (teriparatide) is not only very expensive and difficult to administer but is also associated with side effects including lowered blood pressure, nausea, pain, weakness, and depression. Moreover, the use of teriparatide in rats has been found to cause malignant tumor growth (osteogenic carcinoma). In general, therapeutic choices for osteoporosis are limited and the development of new therapeutic approaches that stimulate bone formation is a priority.

### [Disclosure]

### [Technical Problem]

It is an object of the present invention to provide a composition that has an excellent antioxidant effect and an excellent effect of reducing blood lipid and cholesterol levels, and thus is capable of preventing, alleviating or treating obesity and lipid-related metabolic disease.

Another object of the present invention is to provide a composition capable of preventing, alleviating or treating bone disease by promoting the absorption of calcium and iron.

However, objects which are to be achieved by the present invention are not limited to the above-mentioned objects, and other objects of the present invention will be clearly understood by those skilled in the art from the following description.

### [Technical Solution]

The present inventors have found that the use of a combination of lactic acid bacteria and a vitamin complex has synergistic effects on antioxidant activity, a reduction in blood lipid levels, and the promotion of *in vivo* absorption of calcium and iron, thereby completing the present invention.

In one embodiment, the present invention is directed to a pharmaceutical composition for preventing or treating obesity and lipid-related metabolic disease, which contains, as active ingredients, probiotics and a vitamin complex.

As used herein, the term "probiotics" may be defined as live microbial food supplements which beneficially affect the host by improving the intestinal microbial balance, or more broadly, as living microorganisms, which upon ingestion in certain numbers, exert health effects beyond inherent basic nutrition. Cocktails of various microorganisms, particularly species of *Lactobacillus* and *Bifidobacterium,* have traditionally been used in fermented dairy products to promote health. However to be effective, said probiotics must not only survive manufacturing processing, packaging and storage conditions, but also then must survive transit through the gastrointestinal tract so the probiotic material remains viable to have a positive health effect.

In the present invention, the probiotics lactic acid bacteria strains may be cultured by a general culture method for lactic acid bacteria, and recovered by a separation process such as centrifugation. The recovered lactic acid bacteria may be dried by, but not limited to, freeze-drying, and may be used as probiotics.

The kinds of probiotics that are used in the present invention are not particularly limited. For example, the probiotics may comprise one or more lactic acid bacteria strains selected from the group consisting of *Streptococcus* spp., *Lactococcus* spp., *Enterococcus* spp., *Lactobacillus* spp., *Pediococcus* spp., *Leuconostoc* spp., *Weissella* spp., and *Bifidobacterium* spp. Preferably, the probiotics may comprise *Enterococcus faecium, Lactobacillus rhamnosus,* or a mixture thereof.

In the present invention, the probiotics may be contained in an amount of 3 X 10⁹ to 6 X 10⁹ CFU/g based on the total weight of the composition, but are not limited thereto. If the content of the probiotics is less than 3 X 10⁹ CFU/g, the effect of increasing antioxidant effects cannot be obtained, because the content thereof is low, and if the content of the probiotics is more than 6 X 10⁹ CFU/g, the appearance of the composition can be poor.

Furthermore, the vitamin complex that is used in the present invention may be a mixture of two or more vitamins selected from among water-soluble vitamins, fat-soluble vitamins, or mixtures thereof. Preferably, the vitamin complex may comprise two or more selected from the group consisting of carotenoids (e.g., beta-carotene, zeaxanthin, lutein, lycopene), biotin, choline, inositol, folic acid, pantothenic acid, vitamin A, thiamin (vitamin B1), riboflavin (vitamin B2), niacin (vitamin B3), pyridoxine (vitamin B6), cyanocobalamin (vitamin B12), ascorbic acid (vitamin C), vitamin D, vitamin E, vitamin K, salts thereof, and derivatives thereof, but is not limited thereto.

In the present invention, vitamin A may function as retinoic acid (an irreversibly oxidized form of retinol), which is an important hormone-like growth factor for epithelial cells and the like.

In the present invention, vitamin E may act as a free radical scavenger in cell membranes, erythrocyte membranes and mitochondrial membranes to protect these membranes from oxidative damage, bind to phospholipids and sulfur-containing proteins in the membranes to contribute to membrane stabilization, and exhibit antioxidant activity.

In the present invention, vitamin D is a kind of hormone that is photosynthesized by sunlight, accumulated in calcium *absorbed in vivo,* bone and teeth, and acts on immune cell production in thymus. Moreover, vitamin D has two main forms (vitamin D2 and vitamin D3) that are acquired via dietary intake. Vitamin D acts to *regulate in vivo* cell proliferation and differentiation and immune function and shows anticancer activity, and the main function thereof is to help bone formation by promoting the absorption of calcium salt and phosphate.

In the present invention, vitamin B1 (thiamin) prevents and treats beriberi that is a neurological disease, and vitamin B2 (riboflavin), also called vitamin G, is a component of coenzyme that plays an important role in various metabolisms, and it is involved in a metabolism in which cells are supplied with energy from an energy source. Furthermore, vitamin B6 is composed of three components, pyridoxine, pyridoxal and pyridoxamine, and may be involved in amino acid metabolisms, heme synthesis, carbohydrate metabolisms, neurotransmitter synthesis, vitamin formation, immunometabolism and lipid metabolisms.

In the present invention, vitamin C, an antioxidant substance, can act as a free radical inhibitor, can influence fat metabolism in liver tissue by blocking lipids and oxidative chain reactions, and can remove reactive oxygen species in some circumstances and produce reactive oxygen species in some circumstances. In addition, vitamin C strengthens the immune system and also participates in the formation of connective tissue and supporting tissue to contribute to the maintenance of health of the skin and gum.

In the present invention, the use of the probiotics in combination with the vitamin complex may have a great synergistic effect on antioxidant activity and on a reduction in blood lipid levels, and more specifically, may significantly reduce the levels of blood LDL (low-density lipoprotein) cholesterol, LDL (low-density lipoprotein)/VLDL (very-low-density lipoprotein) cholesterols and triglycerides, thereby preventing or treating obesity and lipid-related metabolic disease.

As used herein, the term "obesity" may refer to a condition or disease with excessive body fat resulting from energy imbalance.

As used herein, the term "lipid-related metabolic disease" refers to a disease caused by excessive lipid accumulation in a living body. Specifically, the lipid-related metabolic disease may be any one selected from the group consisting of diabetes, hyperlipidemia, fatty liver, hepatitis, liver cirrhosis, arteriosclerosis, hypertension, cardiovascular diseases, and metabolic syndromes in which the above-described diseases occur simultaneously.

In another embodiment, the present invention is directed to a pharmaceutical composition for preventing or treating bone disease, which contains, as active ingredient, probiotics and a vitamin complex.

In the present invention, the kinds of probiotics are not particularly limited. For example, the probiotics may comprise one or more lactic acid bacteria strains selected from the group consisting of *Streptococcus* spp., *Lactococcus* spp., *Enterococcus* spp., *Lactobacillus* spp., *Pediococcus* spp., *Leuconostoc* spp., *Weissella* spp., and *Bifidobacterium* spp. Preferably, the probiotics may comprise *Enterococcus faecium, Lactobacillus rhamnosus,* or a mixture thereof.

In the present invention, the probiotics may be contained in an amount of 3 X 10⁹ to 6 X 10⁹ CFU/g based on the total weight of the composition, but are not limited thereto. If the content of the probiotics is less than 3 X 10⁹ CFU/g, the effect of increasing antioxidant effects cannot be obtained, because the content thereof is low, and if the content of the probiotics is more than 6 X 10⁹ CFU/g, the appearance of the composition can be poor.

Furthermore, the vitamin complex that is used in the present invention may be a mixture of two or more vitamins selected from among water-soluble vitamins, fat-soluble vitamins, or mixtures thereof. Preferably, the vitamin complex may comprise two or more selected from the group consisting of carotenoids (e.g., beta-carotene, zeaxanthin, lutein, lycopene), biotin, choline, inositol, folic acid, pantothenic acid, vitamin A, thiamin (vitamin B1), riboflavin (vitamin B2), niacin (vitamin B3), pyridoxine (vitamin B6), cyanocobalamin (vitamin B12), ascorbic acid (vitamin C), vitamin D, vitamin E, vitamin K, salts thereof, and derivatives thereof, but is not limited thereto.

In the present invention, the use of the probiotics in combination with the vitamin complex may have a great synergistic effect on the promotion of *in vivo* absorption of calcium and iron, and thus can prevent or treat various bone diseases.

In the present invention, the bone disease is a disease caused by the imbalance between osteoclasts and osteoblasts in bone, and specific examples thereof include, but are not limited to, osteoporosis, osteomalacia, periodontitis, rheumatoid arthritis, metabolic bone disease, and the like. Preferably, the bone disease may be osteoporosis.

In the present invention, "osteoporosis" is a metabolic disease caused by an overall reduction in bone mass due to the imbalance between bone formation and bone resorption, and is a phenomenon in which bone minerals and proteins are reduced so that bone tissue loose and bone becomes soft. Osteoporosis may be largely divided into postmenopausal osteoporosis and senile osteoporosis. Postmenopausal osteoporosis is caused by an increase in bone resorption due to postmenopausal estrogen deficiency and also by rapid decreases in bone density and bone strength due to decreases in calcium absorption and vitamin D production, and senile osteoporosis is caused mainly by reduced bone mass and calcium absorption resulting from aging and occurs in elderly population without distinction of sex.

As used herein, the term "preventing" may refer to all actions that block symptoms of obesity, lipid-related metabolic disease or bone disease or inhibit or delay the symptoms by use of the pharmaceutical composition of the present invention.

As used herein, the term "treating" may refer to all actions that alleviate or beneficially change symptoms of obesity, lipid-related metabolic disease or bone disease by use of the pharmaceutical composition of the present invention.

In the present invention, the pharmaceutical composition may be in the form of capsules, tablets, granules, injectable solutions, ointments, powders or beverages, and the pharmaceutical composition may be for use in humans.

For use, the pharmaceutical composition of the present invention may be formulated into oral preparations such as powders, granules, capsules, tablets, aqueous suspensions and the like, and forms such as external preparations, suppositories and sterile injectable solutions, according to conventional methods, but is not limited thereto. The pharmaceutical composition of the present invention may contain a pharmaceutically acceptable carrier. For oral administration, the pharmaceutically acceptable carrier may include a binder, a lubricant, a disintegrant, an excipient, a solubilizer, a dispersing agent, a stabilizer, a suspending agent, a coloring agent, a flavoring agent and the like. For injectable preparations, the pharmaceutically acceptable carrier may include a buffering agent, a preservative, an analgesic, a solubilizer, an isotonic agent, a stabilizer and the like. For topical administration, the pharmaceutically acceptable carrier may include a base, an excipient, a lubricant, a preservative and the like. The pharmaceutical composition of the present invention may be formulated into a variety of dosage forms in combination with the aforementioned pharmaceutically acceptable carriers. For example, for oral administration, the pharmaceutical composition may be formulated into tablets, troches, capsules, elixirs, suspensions, syrups, wafers or the like. For injectable administration, the pharmaceutical composition may be formulated as a unit dosage ampoule or a multiple dosage form. In addition, the pharmaceutical composition may also be formulated into solutions, suspensions, tablets, capsules and sustained-release preparations.

Meanwhile, examples of the carrier, excipient, and diluent suitable for the formulation may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, mineral oil or the like. In addition, the pharmaceutical composition of the present invention may further contain a filler, an anti-agglutinating agent, a lubricating agent, a wetting agent, a flavoring agent, an emulsifying agent, a preservative or the like.

Routes of administration of the pharmaceutical composition according to the present invention include, but are not limited to, oral, intravenous, intramuscular, intra-arterial, intramarrow, intrathecal, intracardiac, transdermal, subcutaneous, intraperitoneal, intranasal, intraintestinal, topical, sublingual or rectal routes. Oral or parenteral administration is preferred.

As used herein, the term "parenteral" includes subcutaneous, intracutaneous, intravenous, intramuscular, intraarticular, intrasynovial, intrasternal, intrathecal, intralesional, and intracranial injection or infusion techniques. The pharmaceutical composition of the present invention may also be administered in the form of suppositories for rectal administration.

The pharmaceutical composition of the present invention may vary depending on various factors, including the activity of a particular compound used, the patient's age, weight, general health, sex, diet, administration time, administration mode, excretion rate, drug combination and the severity of a particular disease to be prevented or treated. The dose of the pharmaceutical composition may be suitably selected by a person skilled in the art depending on the patient's condition, weight, the severity of the disease, the type of drug, administration mode and period, and the pharmaceutical composition may be administered at a dose of 0.0001 to 50 mg/kg or 0.001 to 50 mg/kg per day. The pharmaceutical composition may be administered once or several times per day. The dose does not limit the scope of the present invention in any way. The pharmaceutical composition according to the present invention may be formulated as pills, coated tablets, capsules, liquids, gels, syrups, slurries or suspensions.

In still another embodiment, the present invention is directed to a food composition for preventing or alleviating obesity or lipid-related metabolic disease, which contains, as active ingredients, probiotics and a vitamin complex.

In the food composition of the present invention, the contents regarding the above-described probiotics, vitamin complex, obesity and lipid-related metabolic disease are as described above with respect to the pharmaceutical composition, and thus the specific description thereof will be omitted below.

In still another embodiment, the present invention is directed to a food composition for preventing or alleviating bone disease, which contains, as active ingredients, probiotics and a vitamin complex.

In the food composition of the present invention, the contents regarding the above-described probiotics, vitamin complex, bone disease are as described above with respect to the pharmaceutical composition, and thus the specific description thereof will be omitted below.

As used herein, the term "alleviating" may refer to all actions that alleviate or beneficially change symptoms of obesity, lipid-related metabolic disease and bone disease by use of the pharmaceutical composition of the present invention.

The food composition comprising the composition of the present invention as an active ingredient may be prepared as various foods, for example, beverages, gums, teas, vitamin complexes, powders, granules, tablets, capsules, confectionery, cakes, bread and the like. The food composition of the present invention comprises a plant extract having little or no toxicity and side effects, and thus can be used with confidence even when it is administered for a long period of time for preventive purposes.

When the composition of the present invention is contained in the food composition, it may be added in an amount of 0.1 to 50 wt% based on the total weight.

When the food composition is prepared as a beverage, there is no particular limitation, except that the beverage contains the food composition at the indicated percentage. The beverage may additionally contain various flavorings or natural carbohydrates, like conventional beverages. Examples of the natural carbohydrates include monosaccharides such as glucose, disaccharides such as fructose, polysaccharides such as sucrose, conventional sugars such as dextrin, cyclodextrin or the like, and sugar alcohols such as xylitol, sorbitol, erythritol or the like. Examples of the flavorings include natural flavorings (thaumatin, stevia extracts, such as rebaudioside A, glycyrrhizin, etc.) and synthetic flavorings (saccharin, aspartame, etc.).

In addition, the food composition of the present invention may contain various nutrients, vitamins, minerals (electrolytes), flavorings such as synthetic flavorings and natural flavorings, colorants, pectic acid and its salt, alginic acid and its salt, organic acids, protective colloidal thickeners, pH adjusting agents, stabilizers, preservatives, glycerin, alcohol, carbonizing agents that are used in carbonated beverages, etc.

Such components may be used individually or in combination. Although the percentage of such additives is not of great importance, it is generally selected in a range of 0.1 to about 50 parts by weight based on 100 parts by weight of the food composition of the present invention.

In addition, the food composition of the present invention can be administered orally as a food or nutritional product, such as milk or whey-based fermented dairy product, or as a food supplement or a health functional food. Specifically, foods such as milk-based products, beverages, juices, soups, or foods for children may be exemplified, but are not limited thereto. The "milk-based product" means any liquid or semi-solid milk or whey-based product having a varying fat content. The milk-based product can be, for example, cow's milk, goat's milk, sheep's milk, cream, full-fat milk, whole milk, low-fat milk or skim milk, ultrafiltered milk, diafiltered milk, microfiltered milk, milk recombined from powdered milk or whey through any processing, or a processed product, such as yoghurt, curdled milk, sour milk, sour whole milk, butter milk, other fermented milk products, such as viili, filling of snack bars, etc. Another important group includes milk beverages, such as whey beverages, fermented milks, condensed milks, infant or baby milks; icecream; milk-containing food such as sweets.

In still another embodiment, the present invention is directed to a method for preventing or treating obesity and lipid-related metabolic disease, comprising a step of administering to a subject in need of treatment a composition containing probiotics and a vitamin complex.

As used herein, the expression "subject in need of treatment" means a subject having obesity and lipid-related metabolic disease or suspected of having symptoms thereof, in which the lipid-related metabolic disease means a disease caused by excessive lipid accumulation in a living body. Specifically, the lipid-related metabolic disease may be any one selected from the group consisting of diabetes, hyperlipidemia, fatty liver, hepatitis, liver cirrhosis, arteriosclerosis, hypertension, cardiovascular diseases, and metabolic syndromes in which the above-described diseases occur simultaneously, but is not limited thereto.

In the present invention, co-administration of the probiotics with the vitamin complex may have a great synergistic effect on antioxidant activity and on a reduction in blood lipid levels of the subject, and thus can effectively prevent or treat obesity and lipid-related metabolic disease.

In the present invention, the contents regarding the above-described probiotics and vitamin complex are as described above with respect to the pharmaceutical composition, and thus the specific description thereof will be omitted below.

In still another embodiment, the present invention is directed to a method for preventing or treating bone disease, comprising a step of administering to a subject in need of treatment a composition containing probiotics and a vitamin complex.

As used herein, the expression "subject in need of treatment" means a subject having bone disease or suspected of having symptoms thereof, in which the bone disease is a disease caused by the imbalance between osteoclasts and osteoblasts in bone. Specific examples of the bone disease include osteoporosis, osteomalacia, periodontitis, rheumatoid arthritis, metabolic bone disease, and the like. Preferably, the bone disease may be osteoporosis.

In the present invention, the use of the probiotics in combination with the vitamin complex may have a great synergistic effect on the promotion of *in vivo* absorption of calcium and iron, and thus can prevent or treat various bone diseases.

In the present invention, the contents regarding the above-described probiotics and vitamin complex are as described above with respect to the pharmaceutical composition, and thus the specific description thereof will be omitted below.

In the preventing or treating method of the present invention, routes of administration of each composition include, but are not limited to, oral, intravenous, intramuscular, intra-arterial, intramarrow, intrathecal, intracardiac, transdermal, subcutaneous, intraperitoneal, intranasal, intraintestinal, topical, sublingual or rectal routes. Oral or parenteral administration is preferred.

As used herein, the term "parenteral" includes subcutaneous, intracutaneous, intravenous, intramuscular, intraarticular, intrasynovial, intrasternal, intrathecal, intralesional, and intracranial injection or infusion techniques. The composition of the present invention may also be administered in the form of suppositories for rectal administration.

The composition of the present invention may vary depending on various factors, including the activity of a particular compound used, the patient's age, weight, general health, sex, diet, administration time, administration mode, excretion rate, drug combination and the severity of a particular disease to be prevented or treated. The dose of the composition may be suitably selected by a person skilled in the art depending on the patient's condition, weight, the severity of the disease, the type of drug, administration mode and period, and the composition may be administered at a dose of 0.0001 to 50 mg/kg or 0.001 to 50 mg/kg per day. The composition may be administered once or several times per day. The dose does not limit the scope of the present invention in any way.

In the present invention, the composition may be in the form of capsules, tablets, granules, injectable solutions, ointments, powders or beverages, and the composition may be for use in humans.

For use, the composition of the present invention may be formulated into oral preparations such as powders, granules, capsules, tablets, aqueous suspensions and the like, and forms such as external preparations, suppositories and sterile injectable solutions, according to conventional methods, but is not limited thereto. The composition of the present invention may contain a pharmaceutically acceptable carrier. For oral administration, the pharmaceutically acceptable carrier may include a binder, a lubricant, a disintegrant, an excipient, a solubilizer, a dispersing agent, a stabilizer, a suspending agent, a coloring agent, a flavoring agent and the like. For injectable preparations, the pharmaceutically acceptable carrier may include a buffering agent, a preservative, an analgesic, a solubilizer, an isotonic agent, a stabilizer and the like. For topical administration, the pharmaceutically acceptable carrier may include a base, an excipient, a lubricant, a preservative and the like. The composition of the present invention may be formulated into a variety of dosage forms in combination with the aforementioned pharmaceutically acceptable carriers. For example, for oral administration, the composition may be formulated into tablets, troches, capsules, elixirs, suspensions, syrups, wafers or the like. For injectable administration, the composition may be formulated as a unit dosage ampoule or a multiple dosage form. In addition, the composition may also be formulated into solutions, suspensions, tablets, capsules and sustained-release preparations.

Meanwhile, examples of the carrier, excipient, and diluent suitable for the formulation may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, mineral oil or the like. In addition, the composition of the present invention may further contain a filler, an anti-agglutinating agent, a lubricating agent, a wetting agent, a flavoring agent, an emulsifying agent, a preservative or the like.

### [Advantageous Effects]

The composition that is provided according to the present invention has a very excellent antioxidant effect and a very excellent effect of reducing blood lipid levels, and thus can effectively prevent, alleviate or treat obesity and lipid-related metabolic diseases, particularly diseases such as hyperlipidemia.

Moreover, the composition that is provided according to the present invention has a very excellent effect on *in vivo* absorption of calcium and iron, and thus can effectively prevent, alleviate or treat bone diseases, particularly osteoporosis.

### [Description of Drawings]

FIG. 1 graphically shows a change in the weight of each white rat administered with probiotics and a vitamin complex, measured in Test Example 1.
FIG. 2 graphically shows a change in the food efficiency ratio (FER) of each white rat administered with probiotics and a vitamin complex, measured in Test Example 1.
FIG. 3 graphically shows a change in the serum Fe level of each white rat administered with probiotics and a vitamin complex, measured in Test Example 2.
FIG. 4 graphically shows a change in the serum Ca level of each white rat administered with probiotics and a vitamin complex, measured in Test Example 2.
FIG. 5 graphically shows a change in the serum CHOL (total cholesterol) level of each white rat administered with probiotics and a vitamin complex, measured in Test Example 2.
FIG. 6 graphically shows a change in the serum vitamin A level of each white rat administered with probiotics and a vitamin complex, measured in Test Example 3.
FIG. 7 graphically shows a change in the serum vitamin C level of each white rat administered with probiotics and a vitamin complex, measured in Test Example 3.
FIG. 8 graphically shows a change in the serum vitamin E level of each white rat administered with probiotics and a vitamin complex, measured in Test Example 3.
FIG. 9 graphically shows the distribution of *L. rhamnosus* in the feces of white rats as a result of each treatment of Example 1, measured in Test Example 4.
FIG. 10 graphically shows the distribution of *E. facecium* in the feces of white rats as a result of each treatment of Example 1, measured in Test Example 4.

### [Mode for Invention]

Hereinafter, the present invention will be described in further detail with reference to examples. It will be obvious to those skilled in the art that these examples are for illustrative purposes only and are not intended to limit the scope of the present invention.

### Examples

### Preparation Example 1: Preparation of Test Animals

In order to verify the effects of a combination of probiotics and a vitamin complex against bone disease, obesity and lipid-related metabolic disease, 4-week-old white rats (male, SD rats, Saeronbio, Korea) were purchased. Two of the animals were housed in each polypropylene cage and acclimated for one week while they were allowed access to feed and water *ad libitum.* In addition, the animals were housed at a temperature of 24±2°C and a relative humidity of 40±20% with a 12-hr light/12-hr dark cycle. During the acclimation period, the health of the rats was checked, and among these rats, animals whose activity was not reduced were selected and used in subsequent tests.

In order to test effects on the prevention and treatment of hyperlipidemia-related lipid metabolism disorder, a feed obtained by adding 2.5% calcium and 10 ppm Fe to 93G-based feed was fed as basal diet to the animals during the test period while the animals were allowed access to water *ad libitum.*

### Example 1: Oral Administration of Probiotics and Vitamin Complex

In order to verify the effects of probiotics and a vitamin complex on ion absorption, lipid metabolism and antioxidant activity in the white rats selected in Preparation Example 1 above, as shown in Table 1 below, each of PBS (G1) which is a negative control, and 5 X 10⁹ CFU of *L. rhamnosus* and *E. facecium* (G2), a vitamin complex (G3), and a mixture of G2 and G3, which are test substances, was administered orally to the white rats for 5 weeks.

**Table 1**

| Group | Substance administered | Dose |
|---|---|---|
| G1 (N=5) | PBS | - |
| G2 (N=5) | *L. rhamnosus* and *E .facecium* | 5 X 10⁹ CFU/head |
| G3 (N=5) | Thiamine hydrochloride (vitamin B1), | B1: 1.2 mg / head, |
| | Riboflavin (vitamin B2), | B2: 1.4 mg / head, |
| | Nicotinamide (vitamin B3), | B3: 15.02 mg / head, |
| | Pyridoxine hydrochloride (vitamin B6), | B6: 1.5 mg / head, |
| | Biotin (vitamin B7), vitamin A, vitamin E, | A: 700.24 µg/head, |
| | L-ascorbic acid (vitamin C) and vitamin D | E: 11.02 mg/head, |
| | | C: 100.12 mg/head, |
| | | D: 5 µg/head |
| G4 (N=5) | G2 + G3 | 5 X 10⁹ CFU/head + 130.96524 mg/head |

### Test Example 1: Changes in Weight and Feed Intake of White Rats

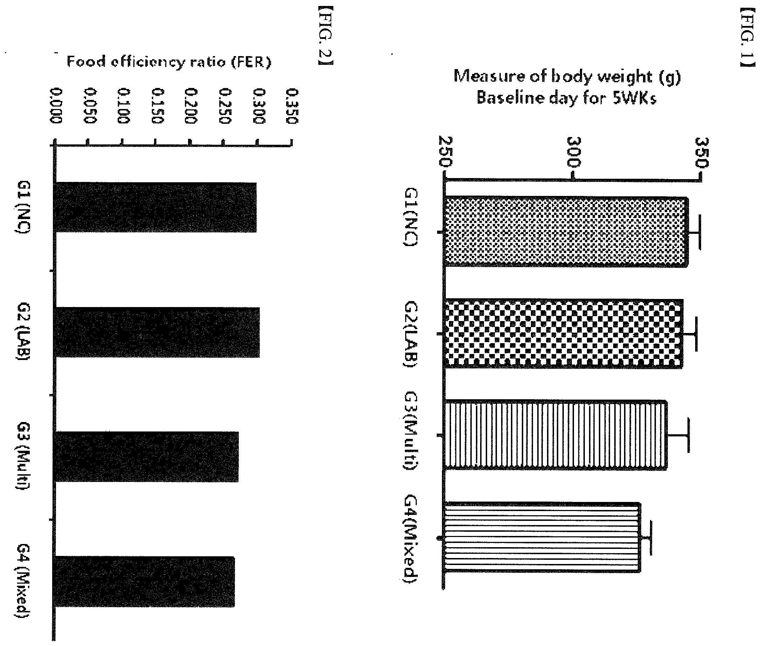

In order to examine the stability and phenotype of each white rat when the probiotics and the vitamin complex were administered to the test animals for 5 weeks in Example 1, the weight change patterns of the white rats during the period from the day before administration to 5 weeks after the start of administration in Example 1 were measured, and the results of the measurement are shown in Table 2 below and FIG. 1. In addition, the food intake of each white rat was measured at an interval of three days, and then the food efficiency ratio (FER) of each white rat was calculated by dividing the weight gain by the food intake. The results of the calculation are shown in Table 3 below and FIG. 2.

**Table 2**

| | | Weight change | | | | | |
|---|---|---|---|---|---|---|---|
| | | 0 week | 1 week | 2 weeks | 3 weeks | 4 weeks | 5 weeks |
| G1 | Mean (g) | 131.8 | 167.4 | 215.5 | 260.2 | 298.8 | 344.3 |
| (NC) | STD (g) | 2.8 | 3.9 | 4.1 | 4.5 | 2.3 | 10.3 |
| G2 (LAB) | Mean (g) | 133.7 | 165.0 | 208.4 | 254.2 | 295.4 | 342.5 |
| | STD (g) | 3.7 | 4.1 | 1.8 | 1.3 | 4.2 | 10.8 |
| G3 (Multiple vitamins) | Mean (g) | 138.4 | 184.0 | 229.3 | 279.4 | 324.8 | 336.4 |
| | STD (g) | 1.6 | 4.6 | 7.5 | 8.7 | 12.7 | 14.6 |
| G4 (Mixed) | Mean (g) | 137.7 | 172.5 | 216.8 | 253.3 | 305.8 | 326.2 |
| | STD (g) | 3.0 | 17.0 | 4.6 | 13.5 | 7.0 | 8.8 |

**Table 3**

| | Number (N) | Food intake (g/week) | Weight gain (g/week) | FER |
|---|---|---|---|---|
| G1 (NC) | 5 | 142.3±1.9 | 42.5±6.5 | 0.299 |
| G2 (LAB) | 5 | 137.4±1.9 | 41.8±6.2 | 0.304 |
| G3 (Multiple vitamins) | 5 | 145±2.0 | 39.6±16.7 | 0.273 |
| G4 (Mixed) | 5 | 141.5±1.7 | 37.7±12.5 | 0.266 |

As shown in Tables 2 and 3 above and FIGS. 1 and 2, the group administered with the vitamin complex or a combination of the probiotics and the vitamin complex showed a slight reduction in weight compared to the group administered with the probiotics alone, but there was no great difference in the food efficiency ratio between the groups. Thus, it can be seen that co-administration of the probiotics and the vitamin complex according to the present invention is not toxic to individuals, like when the probiotics and the vitamin complex were administered alone.

### Test Example 2: Change in Biological Components in Sera of White Rats

The probiotics and the vitamin complex were administered to the rats for 5 weeks in Example 1, and then the rats were fasted for 14 hours, and blood was collected from the abdominal aorta under carbon dioxide anesthesia. The collected blood was allowed to stand at room temperature for 30 minutes, and then centrifuged at 3,000 RPM for 30 minutes to separate sera, and the sera were stored at -80°C.

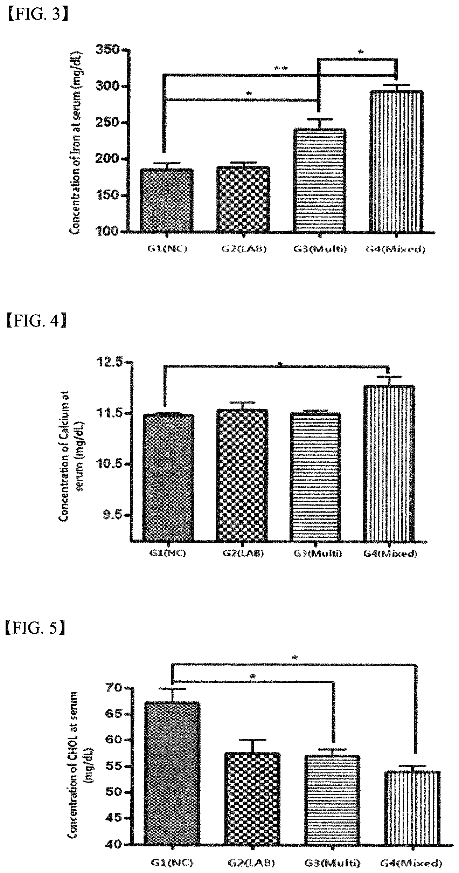

In order to examine effects on the treatment of bone disease, changes in the Fe and Ca levels in the separated sera were measured using an automatic biochemical analyzer (Modular Analytics, Roche, Germany). The results of the measurement are shown in Tables 4 and 5 below and FIGS. 3 and 4. In addition, in order to confirm the effect of reducing blood lipid levels, changes in the serum total cholesterol (CHOL) levels were measured, and the results are shown in Table 6 below and FIG. 5.

**Table 4**

| | Mean ± SD | | | | *P* Value |
|---|---|---|---|---|---|
| Fe (µg/dL) | G1 (NC) | G2 (LAB) | G3 (Multi Vit.) | G4 (mixed) | |
| | 184.3±17.33 | 188.3±10.5 | 240.7±25.01 | 293.3±14.15 | <0.0001 |

**Table 5**

| | Mean ± SD | | | | *P* Value |
|---|---|---|---|---|---|
| Ca (µg/dL) | G1 (NC) | G2 (LAB) | G3 (Multi Vit.) | G4 (mixed) | |
| | 11.47±0.058 | 11.57±0.252 | 11.50±0.100 | 12.03±0.322 | 0.0363 |

**Table 6**

| | Mean ± SD | | | | *P* Value |
|---|---|---|---|---|---|
| CHOL(mg/dL) | G1 (NC) | G2 (LAB) | G3 (Multi Vit.) | G4 (mixed) | |
| | 67.25±5.99 | 57.5±5.06 | 57±2.58 | 54±2.16 | <0.0026 |

As can be seen in Tables 4 and 5 above and FIGS. 3 and 4, calcium and iron levels in the serum of the group administered with a combination of the probiotics and the vitamin complex according to the present invention significantly increased compared to those of the groups administered with the probiotics and the vitamin complex alone.

In addition, as can be seen in Table 6 above and FIG. 5, the blood cholesterol levels were significantly reduced when the probiotics and the vitamin complex according to the present invention were administered in combination compared to when the probiotics and the vitamin complex were administered alone.

Thus, it can be seen that the use of the probiotics in combination with the vitamin complex in the present invention has difficult-to-predict synergistic effects on the promotion of *in vivo* absorption of calcium and iron and on a reduction in blood lipid levels, unlike the probiotics and the vitamin complex are used alone.

### Test Example 3: Examination of Absorption Rate of Vitamin Complex in Serum of White Rats

In order to examine the absorption rate of the vitamin complex in serum, the probiotics and the vitamin complex were administered to the rats for 5 weeks in Example 1, and then the rats were fasted for 14 hours, and blood was collected from the abdominal aorta under carbon dioxide anesthesia. The collected blood was allowed to stand at room temperature for 30 minutes, and then centrifuged at 3,000 RPM for 30 minutes to separate sera, and the sera were stored at -80°C.

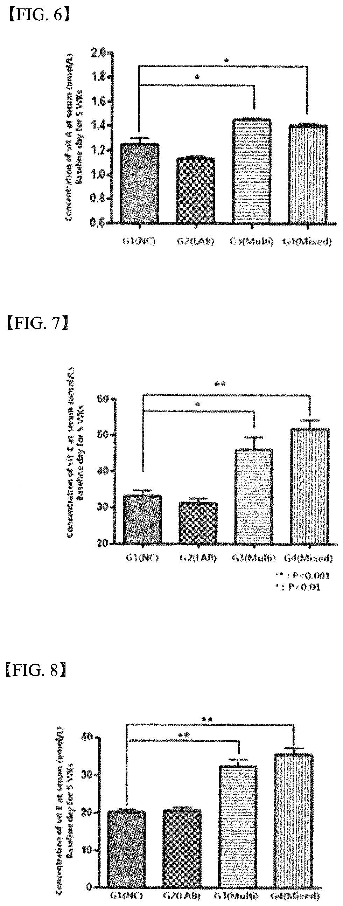

Moreover, in order to examine the absorption rate of the vitamin complex in blood, the levels of vitamin A, C and E in the serum were measured by high-performance liquid chromatography (HPLC). The results of the measurement are shown in Tables 7 to 9 below and FIGS. 6 to 8.

**Table 7**

| | Mean ± SD | | | | *P* Value |
|---|---|---|---|---|---|
| Vit. A µmol/L) | G1 (NC) | G2 (LAB) | G3 (Multi Vit.) | G4 (mixed) | |
| | 1.250±0.090 | 1.135±0.031 | 1.450±0.017 | 1.403±0.035 | <0.0001 |

**Table 8**

| | Mean ± SD | | | | *P* Value |
|---|---|---|---|---|---|
| Vit. C (µmol/L) | G1 (NC) | G2 (LAB) | G3 (Multi Vit.) | G4 (mixed) | |
| | 33.08±2.998 | 31.08±2.354 | 45.93±6.846 | 51.75±4.706 | <0.0001 |

**Table 9**

| | Mean ± SD | | | | *P* Value |
|---|---|---|---|---|---|
| Vit. E (µmol/L) | G1 (NC) | G2 (LAB) | G3 (Multi Vit.) | G4 (mixed) | |
| | 20.18±1.228 | 20.59±1.351 | 32.33±2.950 | 35.58±3.230 | <0.0001 |

As shown in Tables 7 to 9 above and FIGS. 6 to 8, the levels of vitamins in the serum significantly increased when the probiotics and the vitamin complex according to the present invention were administered in combination compared to when the probiotics and the vitamin complex were administered alone.

Thus, it can be seen that the use of the probiotics in combination with the vitamin complex in the present invention has difficult-to-predict synergistic effects on the promotion of *in vivo* absorption of vitamins, unlike the probiotics and the vitamin complex are used alone.

### Test Example 4: Change in Distribution of Lactic Acid Bacteria in Intestine of White Rats

In order to examine the change in intestinal microorganisms of white rats by administration of the probiotics and the vitamin complex according to the present invention, lactic acid bacterial gene expressions in the intestinal microorganisms of G1 to G4 test groups prepared in Example 1 were measured.

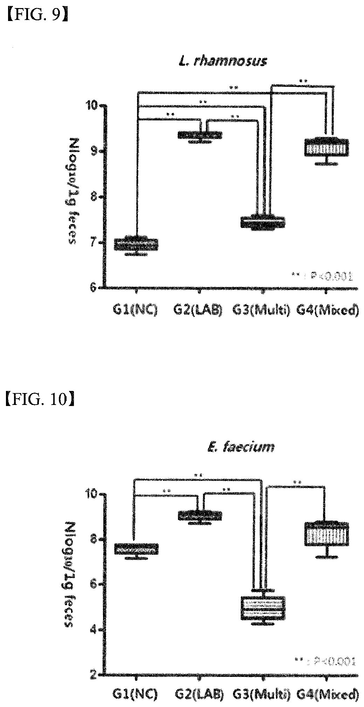

Before oral administration and at 5 weeks after oral administration in Example 1, intestinal feces were isolated from groups G1 to G4, and DNA was extracted using an MP bio stool kit according to the manufacturer's protocol. Using the extracted DNA, real-time polymerase chain reaction (PCR) was performed using primers specific for *L. rhamnosus* and *E. faecium.* The results are shown in Tables 10 and 11 below and FIGS. 9 and 10.

**Table 10**

| | Mean ± SD/g feces | | | | *P* Value |
|---|---|---|---|---|---|
| *L. rhamnosus* | G1 (NC) | G2 (LAB) | G3 (Multi Vit.) | G4 (mixed) | |
| | 6.946±0.1378 | 9.354±0.07893 | 7.426±0.1115 | 9.106±0.2181 | <0.0001 |

**Table 11**

| | Mean ± SD/g feces | | | | P Value |
|---|---|---|---|---|---|
| *E .facecium* | G1 (NC) | G2 (LAB) | G3 (Multi Vit.) | G4 (mixed) | |
| | 7.572±0.2435 | 9.038±0.2018 | 4.938±0.5585 | 8.294±0.6248 | <0.0001 |

As can be seen in Tables 10 and 11 above and FIGS. 9 and 10, the distribution of intestinal lactic acid bacteria significantly increased when the probiotics and the vitamin complex according to the present invention were administered in combination compared to when the probiotics and the vitamin complex were administered alone.

Although the preferred embodiments of the present invention have been described in detail above, it will be obvious to those skilled in the art that the scope of the present invention is not limited to these embodiments and that various modifications and changes are possible without departing from the technical spirit of the present invention as defined in the appended claims.

## Claims

1. A composition for use in a method of preventing or treating obesity and lipid-related metabolic disease, which contains, as active ingredients, probiotics and a vitamin complex.

2. The composition of claim 1, wherein the probiotics comprise one or more lactic acid bacteria strains selected from the group consisting of *Streptococcus* spp., *Lactococcus* spp., *Enterococcus* spp., *Lactobacillus* spp., *Pediococcus* spp., *Leuconostoc* spp., *Weissella* spp., and *Bifidobacterium* spp.

3. The composition of claim 1, wherein the probiotics comprise *Euterococcus faecium, Lactobacillus rhamnosus,* or a mixture thereof.

4. The composition of claim 1, wherein the probiotics are contained in an amount of 3 X 10⁹ to 6 X 10⁹ CFU/g based on the total weight of the composition.

5. The composition of claim 1, wherein the vitamin complex comprises two or more selected from the group consisting of carotenoids, biotin, choline, inositol, folic acid, pantothenic acid, vitamin A, vitamin B1, vitamin B2, vitamin B3, vitamin B6, vitamin B12, vitamin C, vitamin D, vitamin E, vitamin K, salts thereof, and derivatives thereof.

6. The composition of claim 1, wherein the lipid-related metabolic disease is any one selected from the group consisting of diabetes, hyperlipidemia, fatty liver, hepatitis, liver cirrhosis, arteriosclerosis, hypertension, cardiovascular diseases, and metabolic syndromes in which the diseases occur simultaneously.

7. The composition of claim 1, wherein the composition is for oral administration.

8. The composition of claim 1, wherein the composition is a food composition.

9. A composition for use in a method of preventing or treating bone disease, which contains, as active ingredients, probiotics and a vitamin complex.

10. The composition of claim 9, wherein the probiotics comprise one or more lactic acid bacteria strains selected from the group consisting of *Streptococcus* spp., *Lactococcus* spp., *Enterococcus* spp., *Lactobacillus* spp., *Pediococcus* spp., *Leuconostoc* spp., *Weissella* spp., and *Bifidobacterium* spp.

11. The composition of claim 9, wherein the probiotics comprise *Enterococcus faecium, Lactobacillus rhamnosus,* or a mixture thereof.

12. The composition of claim 9, wherein the probiotics are contained in an amount of 3 X 10⁹ to 6 X 10⁹ CFU/g based on the total weight of the composition.

13. The composition of claim 9, wherein the vitamin complex comprises two or more selected from the group consisting of carotenoids, biotin, choline, inositol, folic acid, pantothenic acid, vitamin A, vitamin B1, vitamin B2, vitamin B3, vitamin B6, vitamin B12, vitamin C, vitamin D, vitamin E, vitamin K, salts thereof, and derivatives thereof.

14. The composition of claim 9, wherein the bone disease is any one selected from the group consisting of osteoporosis, osteomalacia, periodontitis, rheumatoid arthritis, and metabolic bone disease.

15. The composition of claim 1, wherein the composition is for oral administration or a food composition.
